# EUROPEAN PATENT APPLICATION

(11) **EP 0 736 534 A1**
(43) Date of publication of application: **09.10.1996**
(21) Application number: 96107729.4
(22) Date of filing: 09.12.1994
(51) Int. Cl.: C07D 487/08, A61K 31/44

(54) **Process for preparing epibatidine**

(30) Priority: 09.12.1993 HU 3500593; 06.10.1994 HU 3500593
(62) Divisional of application: 94119544.8
(71) Applicant: EGIS GYOGYSZERGYAR RT., H-1106 Budapest (HU)
(72) Inventor: Szántay, Csaba, Dr., 1113 Budapest (HU); Balogh née kardos, Zsuzsanna, Dr., 1125 Budapest (HU); Moldvai, István, Dr., 1212 Budapest (HU); Temesvári née Major, Eszter, Dr., 1116 Budapest (HU); Szántay Jr., Csaba, Dr., 1113 Budapest (HU); Mándi, Attila, Dr., 1026 Budapest (HU); Blask , Gábor, Dr., 1113 Budapest (HU); Simig, Gyula, Dr., 1126 Budapest (HU); lax, Györgyi, Dr., 1141 Budapest (HU); Drabant, Sándor, Dr., 1171 Budapest (HU); Szállási, Tamás, Dr., 1027 Budapest (HU); Fekete, Márton, Dr., 1027 Budapest (HU); Gigler, Gábor, 1067 Budapest (HU)
(74) Representative: Beszédes, Stephan G., Dr.

(57) **Abstract**

The subject-matter of the invention is a process for preparing racemic or optically active epibatidine of formula 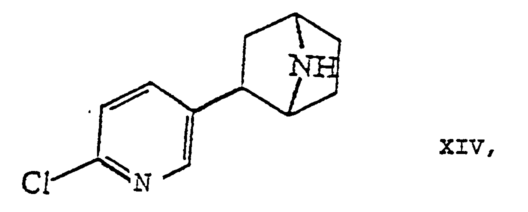 which comprises cyclising a 1-[diacylamino]-2-[6'-chloro)-pyrid-3'-yl]-4-[amino]-cyclohexane of general formula 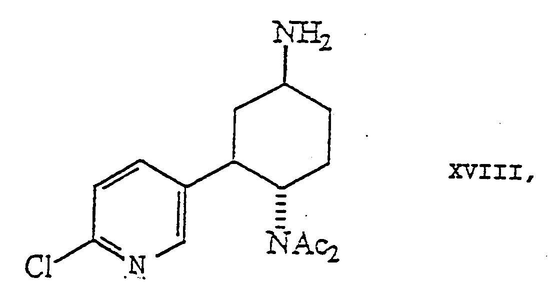 wherein
- Ac: stands for an acyl group.

The advantage of the process is that readily available starting materials are used and the procedure is suitable for industrial scale productions, too.

## Description

The invention is concerned with a process for preparing an organic compound, namely epibatidin, i. e. 2-[6'-(chloro)-pyrid-3'-yl]-7-azabicyclo[2.2.1]heptane, of formula 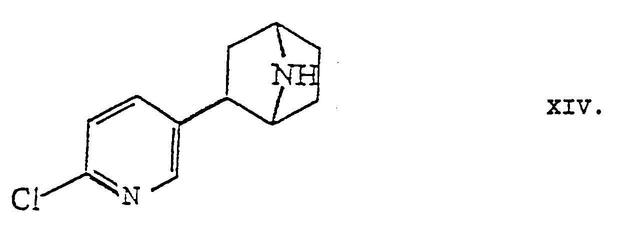 Epibatidine is a minor alkaloid isolated from the skin extracts of an Ecuadoran frog, Epipedobates tricolor, of the familiy Dendrobatidae. Considering the chemical structure it is the first natural substance containing a 7-azabicyclo[2.2.1]heptane skeleton. To the exo position of the said skeleton a 2-(chloro)-pyrid-3-yl substituent is attached, which can rarely be found in nature. The alkaloid possesses a valuable biological property, namely it has 200 times the analgesic potency of morphine. The mechanism of the analgesic effect is different from that of morphine, as it does not sub-side upon administering morphine antagonists, e.g. 17-(allyl)-4,5α-(epoxy)-3,14-di-(hydroxy)-morphinan-6-one [naloxone] (T. F. Spande, H. M. Garraffo, M. W. Edwards, H. J. C. Yeh, L. Pannell, J. W. Daly: J. Am. Chem. Soc., 1992, 114, 3 475 to 3 478).

Several processes have been disclosed in the literature for the synthetic preparation of epibatidine:
A) Ch. A. Broka: Tetrahedron Letters, 34 [1993], 3 251 to 3 254. In this reaction an adduct was prepared by the Diels-Alder reaction of enal obtained by the homologization of 6-(chloro)-nicotinic aldehyde and 2-(trimethylsilyloxy)-1,3-butadiene, it was then reduced with lithium, sodium and potassium tri-2-butyl borohydride [L-selectride®], the hydroxy group of the product was protected by silylation, while the hydroxymethyl substituent was first converted into the hydroxy group in 6 reaction steps and then benzoylated. By selective removal of the protecting groups the dihydroxy derivative was converted into 4-(mesyloxy)-cyclohexylamine containing a 6-(chloro)-pyridyl substituent, and the boiling of the latter compound in dichloromethane resulted in racemic epibatidine.
B) J. W. Daly, T.F. Spande, H. M. Garraffo: US Dept. Health and Human Service, WO 93/18037. The key intermediate of this synthesis is 3-(pyridyl)-2-cyclohexa-1,3-diene, which was prepared from cyclohexane-1,2-dione and converted into Diels-Alder adduct with tert.butylnitrosoformiate. The adduct was hydrogenated catalytically, the thus-obtained aminoalcohol derivative the amino group of which is protected by a tert.-butylformate group was treated with thionyl chloride, the product, the corresponding 1-[pyrid-3'-yl]-2-[chloro]-5-[amino]-cyclohexane protected on the nitrogen atom of the amino group by a tert.-butylformate group, was cyclized with base-treatment to give 1-[pyrid-3'-yl]-7-azabicyclo[2.2.1]heptane protected on its aza-nitrogen atom by a tert.-butylformate group and chlorinated by a photochemical reaction to 1-[pyrid-3'-yl]-7-azabicycl of [2.2.1]heptane protected on its aza-nitrogen atom by a tert.-butylformate group. The hydrolysis of the acid amide bond with trifluoroacetic acid resulted in racemic epibatidine.
C) D. F. Huang, T. Y. Shen: Tetrahedron Letters, 34 [1993], 4 477 to 4 480. An adduct obtained by the Diels-Alder reaction of phenylsulfonyl-6-(chloro)-3-(pyridyl)-acetylene prepared from with N-(carbomethoxy)-pyrrole and 6-(chloro)-nicotinic acid was desulfonated with sodium amalgam concomitantly reducing one of the double bonds of the 7-azanorbornadiene ring, hydrogenated catalytically to a mixture of N-(carbomethoxy)-epi-epibatidine and N-(carbomethoxy)-epibatidine, the protecting group was dehydrolized to obtain racemic epibatidine and racemic epi-epibatidine, which were then separated by column chromatography. The racemic epibatidine was resolved with di-(p-toluyl)-tartaric acid.
D) S. R. Fletcher, R. Baker, M. S. Chambers, S. C. Hobbs, P. J. Mitchell: J. Chem. Soc. Chem. Comm., 1993, 1 216 to 1 218. The key intermediate of this synthesis route is tert.butyloxycarbonyl-7-azabicyclo[2.2.1]heptan-2-one, which was prepared in 7 reaction steps from cyclohexene amine containing a trifluoroacetyl protecting group and reacted with 5-lithio-2-(chloro)-pyridine. From the thus-obtained adduct water was removed and the thus-formed double bond was hydrogenated catalytically to obtain a mixture of racemic epibatidine and epi-epibatidine isomers containing tert.butyloxycarbonyl protecting group. The undesired epimer epi-epipatidine (endo-isomer) protected on the nitrogen atom of the 7-azabicyclo[2.2.1] heptane ring by a tert.-butoxycarbonyl protective group (BOC protective group) was subjected to epimerization by boiling in tert.butanol in the presence of potassium tert.butylate. The racemic epibatidine (exo-isomer) was resolved by applying a chiral HPLC method and by salt formation with a chiral acid.
E) E. J. Corey,* Teck-Peng Loh, Sidduri AchyuthaRao, Donnette C. Daley, and Sepehr Sarshar: Journal of Organic Chemistry, Vol. 58, No. 21, 1993, 5 600 to 5 602. From this it has been known to cyclize 1-[trifluoracetylamino]-2-[6'-(chloro)-pyrid-3'-yl]-4,5-di[bromo]-cyclohexane cyclizing with potassium tert.-butyloxyd in tetrahydrofurane to dextro 5-[bromo]-N-[trifluoroacetyl]-epibatidine, to debrominate the latter to dextro N-(trifluoroacetyl)-epibatidine by treatment with tributyltin and aluminumbornitride in benzene and to deacylate the latter with sodium methylate in methanol to yield (-)-epibatidine.

A serious drawback of the methods known from the literature resides in the cumbersome reaction steps which would make the industrial scale application rather complicated.

A further disadvantage of the known preparation methods resides in the fact that they require the application of difficultly available, expensive and inconvenient substances.

The problem underlying to the invention is to create a novel process for preparing epibatidine, which is devoid of the drawbacks of the hitherto known processes and can be accomplished by using substances readily available and preparable even on an industrial scale and which is also industrially applicable.

The above surprisingly has been attained by the invention.

The subject matter of the invention is a process for preparing racemic or optically active epibatidine of formula 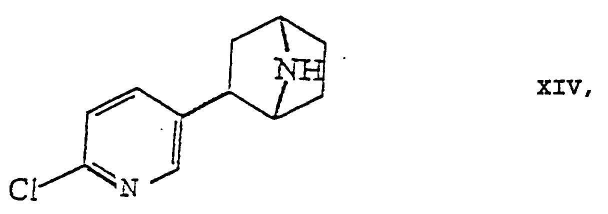 characterized by
a)
   α) selectively reducing the nitro group of 1-[nitro]-2-[6'-(chloro)-pyrid-3'-yl]-cyclohexane-4-one of formula 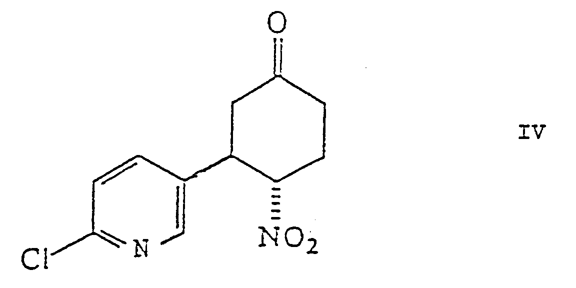 to yield 1-[amino]-2-[6'-(chloro)-pyrid-3'-yl]-cyclohexane-4-one of formula 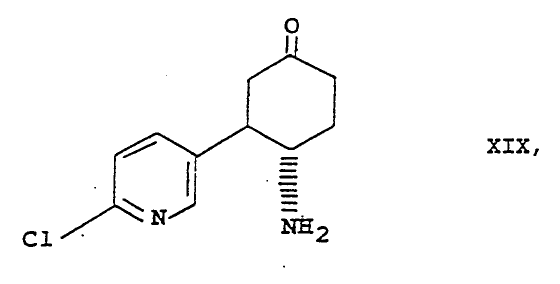
   β) protecting the carbonyl group in the latter, preferably to yield a 1-[amino]-2-[6'-(chloro)-pyrid-3'-yl]-cyclohexane-4-one ketal of general formula 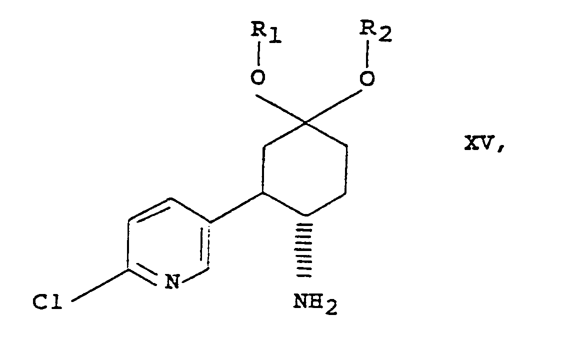 wherein
      - R₁ and R₂: stand for C₁₋₄-alkyl groups or together form a C₂₋₄-alkylene group,
      particularly 1-[amino]-2-[6'-(chloro)-pyrid-3'-yl]-cyclohexane-4-one-ethylene ketal of formula 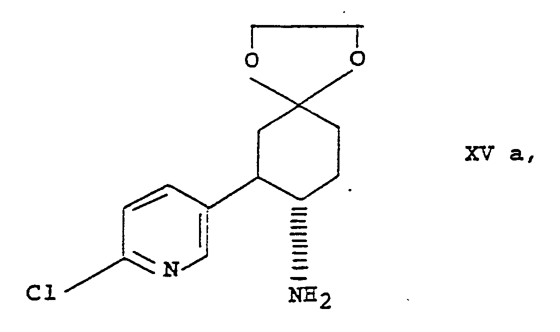 i. e. the compound of formula XV, in which R₁ and R₂ together form an ethylene group,
   γ) diacylating the amino group of the latter, preferably to yield a 1-[diacylamino]-2-[6'-(chloro)-pyrid-3'-yl]-cyclohexane-4-one ketal of general formula 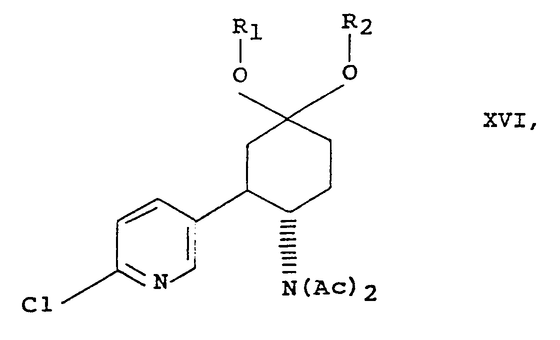 wherein
      - R₁ and R₂: stand for C₁₋₄-alkyl groups or together form a C₂₋₄-alkylene group and
      - Ac: represents an acyl group,
      particularly a 1-[diacylamino]-2-[6'-(chloro)-pyrid-3'-yl]-cyclohexane-4-one-ethylene ketal of formula 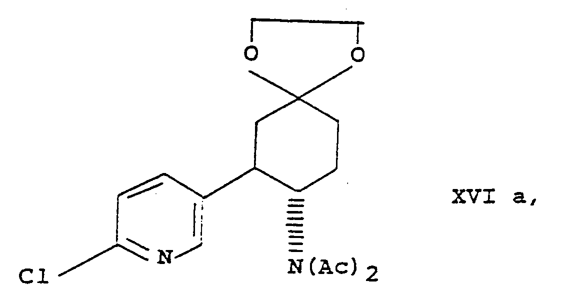 wherein
      - Ac: represents an acyl group,
      i.e. the compound of formula XVI, in which R₁ and R₂ together form an ethylene group,
   δ) deprotecting the oxo group of the latter to yield a 1-[diacylamino]-2-[6'-(chloro)-pyrid-3'-yl]-cyclohexane-4-one of general formula 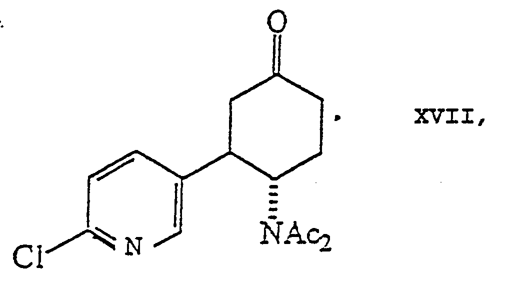 wherein
      - Ac: stands for an acyl group,
   ε) exchanging in the latter the oxo group for an amino group to yield a 1-[diacylamino]-2-[6'-(chloro)-pyrid-3'-yl]-4-[amino]-cyclohexane of general formula 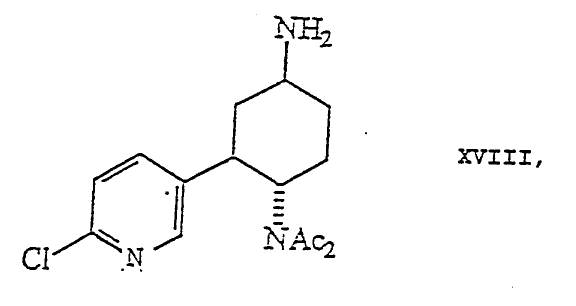 wherein
      - Ac: stands for an acyl group,
      and
   ω) cyclizing the latter into epibatidine of formula XIV
      or
b)
   α) cyclizing 1-[6'-(chloro)-pyrid-3'-yl]-3-[oxo]-6-[nitro]-hexa-1-ene of formula 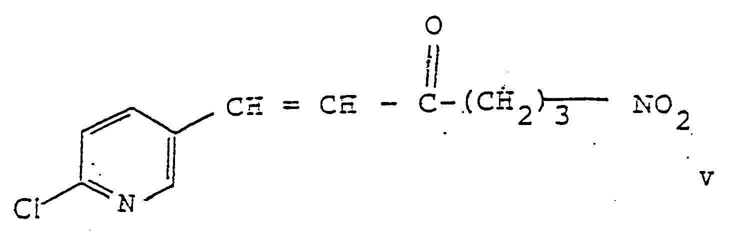 in the presence of an optically active base to optically active 1-[nitro]-2-[6'-(chloro)-pyrid-3'-yl]-cyclohexane-4-one of formula IV
      and thereafter
   β) converting the optically active 1-[nitro]-2-[6'-(chloro)-pyrid-3'-yl]-cyclohexane-4-one of formula IV into an optically active epibatidine of formula XIV analogously to variant a).

If desired, in a manner known per se a thus-obtained racemic epibatidine of formula XIV may be resolved into the optically active isomers and/or the thus-obtained optically active isomers may be racemised or, if desired, a racemic intermediate product may be separated in any stage of the synthesis into the optically active isomers and the thus-obtained optically active isomers may be converted into optically active epibatidine of formula XIV.

As opposed to WO 93/18037 in which for the cyclisation an amino group which is protected by a tert.-butylformate group is present and no chlorine substituent on the pyridine ring in step a) ω) the cyclisation is carried out with a compound in which the amino group is not protected and on the pyridine ring already the chlorine substituent of epibatidine is present.

Analogous is the situation as compared to Journal of Organic Chemistry, Vol. 58, No. 21, 1993, pages 5 600 to 5 602 with the modification that there the protective group on the nitrogen atom of the amino group is a trifluoroacetyl group.

Though in Scheme 2 on page 4 478 of Tetrahedron Letters, Vol. 34, No. 28, pages 4 477 to 4 480, 1993 there is an acetyl group on a nitrogen atom, however, this is on the nitrogen atom of the 7-azabicyclo[2.2.1]heptane ring already formed, whereas in steps a) γ), a) δ) and a) ε) of the process accordinc to the invention 2 acyl groups are attached to a nitrogen atom of an amino group attached to a cyclohexane ring which reacts with an amino group of the same cyclohexane ring. Hence the mutual reactions are entirely different.

Contrary to the process according to J. Chem. Soc. Chem. Comm., 1993, 1 216 to 1 218 in which the epimerization to epibatidine is carried out on an epi-epibatidine protected on the nitrogen atom of the 7-azabicycio[2.2.1]heptane ring by a Boc protective group whereas in variant a) of the process according to the invention the epimerization is carried out on unprotected epi-epibatidine itself.

The epibatidine of formula XIV has a chiral structure so that it can be present in racemic or optically active form. The invention encompasses the preparation of both the racemic and the optically active epibatidine of formula XIV.

The racemic epibatidine of formula XIV can optionally be resolved. The resolution can be carried out by methods knob per se. One can proceed e. g. by reacting the racemic epibatidine of general formula XIV with an optically active acid, such as tartaric acid, di-(0,0'-p-toluyl)-tartaric acid or dibenzoyltartaric acid, separating the thus-obtained diastereoisomeric salt pair, e. g. by fractional crystallization and liberating the desired optically active epibatidine of formula XIV from the salt thereof.

In variant a), part α) of the process according to the invention the selective reduction of the nitro group of 1-[nitro]-2-[6'-(chloro)-pyrid-3'-yl]-cyclohexane-4-one of formula IV to yield the 1-[amino]-2-[6'-(chloro)-pyrid-3'-yl]-cyclohexane-4-one of formula XIX may be carried out by catalytic hydrogenation or reduction with a chemical agent.

When a chemical reducing agent is used, preferably the selective reduction of the nitro group of 1-[nitro]-2-[6'-(chloro)-pyrid-3'-yl]-cyclohexane-4-one of formula IV is carried out by Bechamps reduction, or with zinc in glacial acetic acid or with zinc, iron or tin in hydrochloric acid or with stannous (II)-chloride the latter being particularly preferred. One may work preferably by using stannous(II)-chloride in a polar organic solvent, e. g. a C₁₋₄-alkanol or tetrahydrofurane, or a mixture of such. Chemical reduction may be performed in a neutral medium as well. The selective reduction having been completed the reaction mixture may be evaporated.

Preferably in variant a), part β) of the process according to the invention protecting of the carbonyl group in the 1-[amino]-2-[6'-(chloro)-pyrid-3'-yl)-cyclohexane-4-one of formula XIX is made in the form of an ethylene ketale thus yielding 1-[amino]-2-[6'-(chloro)-pyrid-3'-yl]-cyclohexane-4-one ethylene ketal of formula XVa. Advantageously this reaction is carried out by dissolving the evaporation residue above obtained in an apolar organic solvent non miscible with water, e. g. benzene or toluene, benzene being preferred, or a mixture of such, adding a monohydric C₁₋₄-alcohol or dihydric C₂₋₄-alcohol, e. g. methanol, ethanol, ethylene glycol or an ortho formic acid ester, ethylene glycol being preferred, and heating the reaction mixture to boiling in the presence of an inorganic or organic acid or a salt thereof, preferably pyridinium tosylate. The 1-[amino]-2-[6'-(chloro)-pyrid-3'-yl]-cyclohexane-4-one ketal of general formula XV, particularly 1-[amino]-2-[6'-(chloro)-pyrid-3'-yl]-cyclohexane-4-one ethylene ketal of formula XVa, thus obtained can be isolated in a known manner. Thus one may proceed by cooling the reaction mixture, separating the layers, making the benzene phase alkaline, washing with water and saturated brine, drying and evaporating. The ethylene glycol layer is made alkaline, the precipitated product is filtered, to the filtrate water is added and it is extracted with an organic solvent non miscible with water, e. g. a chlorinated hydrocarbon, preferably chloroform. The organic phase is washed, dried and evaporated. The evaporated residue thus obtained is combined with the evaporation residue of the benzene phase. The 1-[amino]-2-[6'-(chloro)-pyrid-3'-yl]-cyclohexane-4-one ketal of general formula XV, particularly 1-[amino]-2-[6'-(chloro)-pyrid-3'-yl]-cyclohexane-4-one ethylene ketal of formula XVa, thus obtained may be purified by column chromatography.

In variant a), part γ) of the process according to the invention preferably the diacylation of the amino group of the 1-[amino]-2-[6'-(chloro)-pyrid-3'-yl]-cyclohexane-4-one ketal of general formula XV is carried out to yield a 1-[diacylamino]-2-[6'-(chloro)-pyrid-3'-yl]-cyclohexane-4-one ketal of general formula XVI, in which Ac stands for C₁₋₄-alkylsulfonyl or arylsulfonyl, particularly methanesulfonyl, p-toluenesulfonyl or p-(bromo)-phenylsulfonyl. Thus it is preferred to use an acylating agent containing a sulfonyl group, e. g. p-toluene sulfonyl chloride, p-(bromo)-phenyl sulfonyl chloride, trifluoromethane sulfonyl chloride or methane sulfonyl chloride. Advantageously this diacylation is carried out by dissolving the 1-[amino]-2-[6'-(chloro)-pyrid-3'-yl]-cyclohexane-4-one ketal of general formula XV in a polar aprotic solvent, e. g. dimethyl formamide, and reacting it with an acylating agent in the presence of an organic base, e. g. triethyl amine and/or sodium hydride, at room temperature. If desired, the diacylation of the amino group may be carried out by first monoacylating and thereafter introducing the second acyl group into the monoacylated product. The 1-[diacylamino]-2-[6'-(chloro)-pyrid-3'-yl]-cyclohexane-4-one ketal of general formula XVI may be isolated from the reaction mixture by methods known per se. Thus one may proceed e. g. by pouring the reaction mixture into water, removing the precipitate by filtration, washing and drying. The 1-[diacylamino]-2-[6'-(chloro)-pyrid-3'-yl]-cyclohexane-4-one ketal of general formula XVI thus obtained may be purified by recrystallization or column chromatography, if desired.

Preferably in variant a), part δ) of the process according to the invention the deprotection of the oxo group of the 1-[diacylamino]-2-[6'-(chloro)-pyrid-3'-yl]-cyclohexane-4-one ketal of general formula XVI to yield a 1-[di-acylamino]-2-[6'-(chloro)-pyrid-3'-yl]-cyclohexane-4-one of general formula XVII is carried out by acidic treatment. This removal of the protecting group of the oxo group may be carried out by methods known per se. Thus the 1-[diacylamino]-2-[6'-(chloro)-pyrid-3'-yl]-cyclohexane-4-one ketal of general formula XVI may be dissolved in an organic solvent with 1 to 4 carbon atom(s) and containing an oxo group, e. g. acetone, and heated to boiling in the presence of an organic or inorganic acid, preferably concentrated hydrochloric acid. One may proceed preferably by continuously distilling off acetone from the system and adding acetone dropwise to the reaction mixture at the same rate as acetone is removed. The 1-[diacylamino]-2-[6'-(chloro)-pyrid-3'-yl]-cyclohexane-4-one of general formula XVII may be isolated in a known manner. Thus one may proceed by cooling and evaporating the reaction mixture and adding a solvent non-miscible with water, e. g. a chlorinated hydrocarbon, preferably chloroform, and a sodium hydroxide solution to the mixture. The layers are separated, the aqueous phase is extracted with chloroform, the combined organic phases are washed, dried and evaporated. The 1-[diacylamino]-2-[6'-(chloro)-pyrid-3'-yl]-cyclohexane-4-one of general formula XVII may be purified by crystallization or column chromatography, if desired.

Preferably in variant a), part ε), of the process according to the invention the exchange of the oxo group of the 1-[diacylamino]-2-[6'-(chloro)-pyrid-3'-yl]-cyclohexane-4-one of general formula XVII for an amino group is carried out by reduction with a complex metal hydride in the presence of ammonium acetate. Particularly lithium cyano borohydride and/or sodium cyanoborohydride is/are used as complex metal hydride(s), above all the latter one. Advantageously this reaction may be performed by dissolving the 1-[diacylamino]-2-[6'-(chloro)-pyrid-3'-yl]-cyclohexane-4-one of general formula XVII in an anhydrous C₁₋₄-alkanol, preferably methanol, and carrying out the reduction in this. The reaction is preferably carried out at room temperature. The 1-[diacylamino]-2-[6'-(chloro)-pyrid-3'-yl]-4-[amino]-cyclohexane of general formula XVIII thus obtained may be isolated in a manner known per se. Thus one may proceed by decomposing the excess of the reducing agent by adding an organic solvent containing an oxo group, e. g. acetone, evaporation the reaction mixture, dissolving the residue in an organic solvent non-miscible with water and washing, drying and evaporating the organic phase. The 1-[diacylamino]-2-[6'-(chloro)-pyrid-3'-yl]-4-[amino]-cyclohexane of general formula XVIII thus obtained may be purified by crystallization or column chromatography, if desired.

Preferably in variant a), part ω) of the process according to the invention the cyclization of the 1-[diacylamino]-2-[6'-(chloro)-pyrid-3'-yl]-4-[amino]-cyclohexane of general formula XVIII, to yield epibatidine of formula XIV is carried out by heating, particularly to boiling, in [an] organic solvent(s). Particularly preferably for the cyclization as organic solvent(s) [an] anhydrous dipolar aprotic solvent(s), most preferably dimethyl formamide, is/are used. Furthermore it is preferred to carry out the cyclisation in the presence of sodium borohydride. The epibatidine of formula XIV thus obtained may be isolated by methods known per se. Thus one may proceed by pouring the reaction mixture into water and separating the epibatidine of formula XIV by filtration or extraction. The epibatidine of formula XIV may be purified by crystallization or column chromatography, if desired.

The optically active forms of epibatidine of formula XIV may be prepared by several methods.

According to variant b) α) of the process according to the invention 1-[6'-(chloro)-pyrid-3'-yl]-3-[oxo]-6-[nitro]-hexa-1-ene of formula V is subjected to cyclisation in the presence of an optically active base to yield an optically active 1-[nitro]-2-[6'-(chloro)-pyrid-3'-yl]-cyclohexane-4-one of formula IV. As optically active base preferably (+)-α-(phenyl)-ethyl-amine or (-)-α-(phenyl)-ethyl-amine is used. The reaction may be carried out in the presence of an inert organic solvent, preferably an ether, e. g. tetrahydrofurane or dioxane, or a mixture of such.

The optically active 1-[nitro]-2-[6'-(chloro)-pyrid-3'-yl]-cyclohexane-4-one of formula IV may be converted into optically active epibatidine of formula XIV in an analogous manner to that disclosed above as variant a) in connection with the preparation of racemic epibatidine of formula XIV this being variant b) β) of the process according to the invention.

The still achiral but prochiral 1-[6'-(chloro)-pyrid-3'-yl]-3-[oxo]-6-[nitro]-hexa-1-ene of formula V may be converted into an optically active 1-[nitro]-2-[6'-(chloro)-pyrid-3'-yl]-cyclohexane-4-one of formula IV by means of an enantioselective synthesis. The optically active 1-[nitro]-2-[6'-(chloro)-pyrid-3'-yl]-cyclohexane-4-one of formula IV can be transformed into a dextro or laevo rotating epibatidine of formula XIV with the aid of the synthesis steps described above as variant a) for the preparation of racemic epibatidine.

Optically active epibatidine of formula XIV can also be prepared by separating racemic epibatidine of formula XIV into the optically active isomers or subjecting a racemic intermediate at a suitable stage of the synthesis to resolution and converting the optically active intermediate thus obtained into optically active epibatidine of formula XIV.

Racemic epi-epibatidine of formula XIII may be subjected to resolution. The process may be carried out by reacting racemic epi-epibatidine of formula XIII with an optically active acid, e. g. optically active tartaric acid, dibenzoyl-tartaric acid or di-0,0'-(p-toluyl)-tartaric acid, separating the diastereoisomeric salts thus formed, e. g. by fractionated crystallization, and liberating the optically active epi-epibatidine of formula XIII from the salt thereof. Then one may proceed to epimerisation of the latter.

According to a further embodiment of the process according to the invention another intermediate of chiral structure is subjected to resolution and the optically active intermediate thus obtained is converted into the desired optically active end-product optically active epibatidine of formula XIV in an analogous manner to the process described above for the preparation of racemic epibatidine of formula XIV.

Thus according to a preferred embodiment of the invention optically active epibatidine of formula XIV may be prepared by carrying out the variant b) β) of the process according to the invention with the optically active 1-[nitro]-2-[6'-(chloro)-pyrid-3'-yl]-cyclohexane-4-one of formula IV as above described.

The starting substance 1-[nitro]-2-[6'-(chloro)-pyrid-3'-yl]-cyclohexanes-4-one of formula IV for variant a) of the process according to the invention and the starting substance 1-[6'-(chloro)-pyrid-3'-yl]-3-[oxo]-6-[nitro]-hexa-1-ene of formula V for variant b) of the process according to the invention could have been prepared as follows.

A particular advantage of the synthesis resides in the fact that 1-(nitro)-pentane-4-one of formula 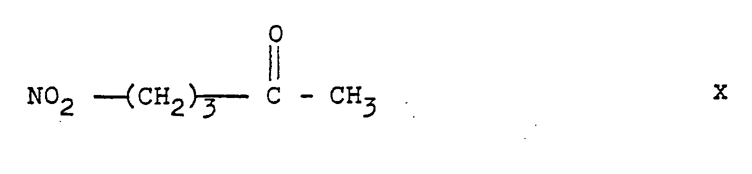 is used as starting substance, which can be obtained by reacting commercially readily available compounds, that is nitromethane of formula

H₃C - NO₂ XII

and methylvinylketone of formula 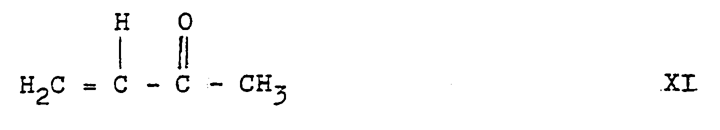 The 1-(nitro)-pentan-4-one of formula X can be produced according to the method of D. E. Bergbreiter and J. J. Lalonde (J. Org. Chem. 52 [1987], 1 601 to 1 603).

In the next reaction step the 1-(nitro)-pentane-4-one of formula X is brominated. Preferably the bromination is carried out with elementary bromine in a lower alcohol, particularly methanol. Conveniently the bromination is performed at a temperature of about room temperature taking care of that the temperature of the reaction mixture should not rise above 40°C. The acetalic ether bond being formed in the reaction is hydrolyzed. The 1-(bromo)-5-(nitro)-pentane-2-one of formula 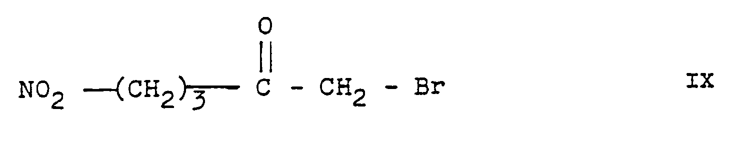 thus obtained may be isolated by extracting the aqueous solution with a solvent non-miscible with water such as chlorinated hydrocarbon, aromatic hydrocarbon, ethyl acetate or preferably ether, washing the extract first acid-free with a caustic solution then neutral with water, dried and evaporated. The 1-(bromo)-5-(nitro)-pentane-2-one of general formula IX is optionally purified by column chromatography.

The 1-(bromo)-5-(nitro)-pentane-2-one of formula IX is then reacted with a triaryl phosphine to obtain a phosphonium salt [5-(nitro)-pentane-2-one]-triaryl-phosphonium bromide of general formula 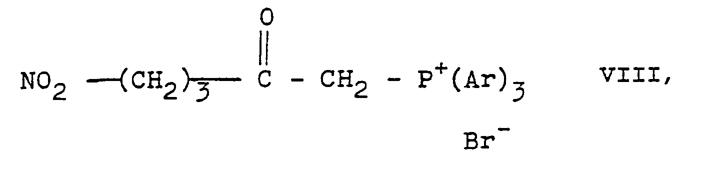 wherein
- Ar: stands for an aryl group, preferably phenyl group.
The reaction is preferably carried out with triphenyl phosphine. It is advantageously performed in an apolar aprotic solvent, preferably an aromatic hydrocarbon, particularly benzene or a mixture of such. It is preferable to carry out the reaction by adding to the solution of the 1-(bromo)-5-(nitro)-pentane-2-one of formula IX in an apolar aprotic solvent a solution of triphenyl phosphine in the same solvent. The reaction may be carried out at a temperature of from 10 to 30°C, preferably at room temperature. The oily product gets crystalline upon standing. The crystalline [5-(nitro)-pentane-2-one]-triaryl-phosphonium bromide of general formula VIII can be filtered off and washed.

The phosphonium salt [5-(nitro)-pentane-2-one]-triaryl-phosphonium bromide of general formula VIII is then converted into a [5-(nitro)-pentane-2-one]-triaryl-phosphorane, preferably [5-(nitro)-pentane-2-one]-triphenyl-phosphorane, of general formula 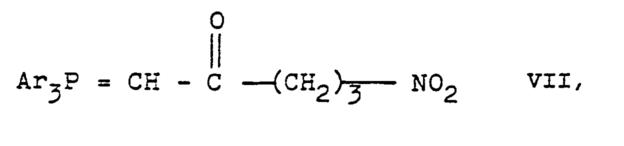 wherein
- Ar: stands for an aryl group, preferably phenyl group.
The phosphonium salt [5-(nitro)-pentane-2-one]-triaryl-phosphoniun bromide is dissolved in an apolar aprotic solvent non-miscible with water, preferably in a halogenated aliphatic hydrocarbon, such as dichloromethane, or a mixture of such and stirred with a diluted alkali hydroxide solution, e. g. sodium or potassium hydroxide solution, at a temperature of about room temperature. The phases are then separated, the organic layer is washed, dried and evaporated.

The [5-(nitro)-pentane-2-one]-triaryl-phosphorane, preferably [5-(nitro)-pentane-2-one]-triphenyl-phosphorane, of general formula VII thus obtained is reacted with 6-(chloro)-pyridine-3-aldehyde [6-(chloro)-nicotinic aldehyde] of formula 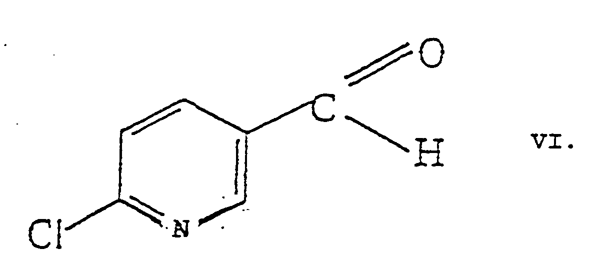 The reaction can be performed in an anhydrous aprotic solvent, preferably a halogenated aliphatic hydrocarbon, such as dichloromethane, or a mixture of such. The reaction can be carried out under heating, preferably at the boiling point of the reaction mixture. It is preferable to perform the reaction by adding to the solution of the [5-(nitro)-pentane-2-one]-triaryl-phosphorane, preferably [5-(nitro)-pentane-2-one]-triphenyl-phosphorane, of general formula VII in an anhydrous aprotic solvent the solution of the 6-(chloro)-pyridine-3-aldehyde of formula VI in the same solvent. The reaction mixture is then preferably worked up by cooling, washing and evaporating. The olefine 1-[6'-(chloro)-pyrid-3'-yl]-3-(oxo)-6-(nitro)-hexa-1-ene of formula 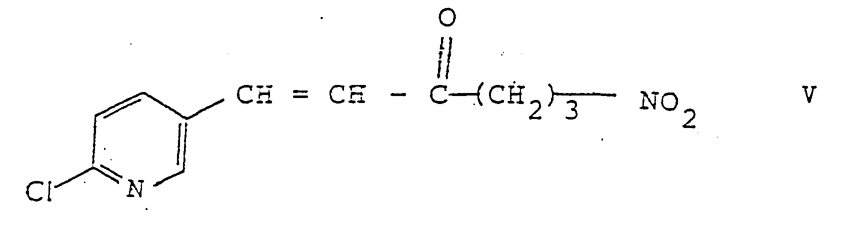 thus obtained can be purified by crystallization or column chromatography.

The 6-(chloro)-pyridine-3-aldehyde of formula VI could be prepared readily from the commercially available 6-(chloro)-nicotinic acid by methods known from the literature (F. E. Ziegler, J. G. Sweeny: Tetrahedron Letters, [1969], 1 097 to 1 110).

The olefine 1-[6'-(chloro)-pyrid-3'-yl]-3-(oxo)-6-(nitro)-hexa-1-ene of formula V obtained as specified above then may be subjected to cyclization. This reaction may be carried out in an anhydrous aprotic organic solvent. As reaction medium preferably cyclic ethers, e. g. tetrahydrofuran, or a mixture of such can be applied. The cyclization is preferably carried out in the presence of a base, particularly potassium fluoride applied onto a basic aluminum oxide carrier (D. E. Bergbreiter, J. J. Lalonde: J. Org. Chem. 52 [1987], 1 601 to 1 603). The cyclization may be carried out at a temperature of about room temperature. The thus-obtained nitroketone 1-[nitro]-2-[6'-(chloro)-pyrid-3'-yl]-cyclohexane-4-one of formula IV may be isolated by removing the base and evaporating the solution. Optionally the 1-[nitro]-2-[6'-(chloro)-pyrid-3'-yl]-cyclohexane-4-one of formula IV thus obtained can be purified by crystallization or column chromatography.

The invention is illustrated in detail with the aid of the following Examples. In these the ratios of the components of the mixtures for chromatography are always by volume. Moreover in the following Examples unless other indications have been made column chromatography is to be meant as having been carried out on silica gel with the designation "Kieselgel 60" (0.063 to 0.200 mm). Furthermore "brine" is a saturated sodium chloride solution. For the potassium fluoride precipitated on aluminum oxide always the total amount of these 2 substances and the potassium fluoride contents thereof have been indicated.

In a manner known per se the free epibatidine of formula XIV can be converted into an acid addition salt, particularly hydrochloride salt, and/or the free epibatidine of formula XIV can be liberated from an acid addition salt of it and/or converted into another acid addition salt thereof.

### Example 1

### (±)-Epibatidine

### Step 1

### (±)-1α-[Amino]-2β-[6'-(chloro)-pyrid-3'-yl]-cyclohexane-4-one

2.54 g (0.01 mole) of (±)-1α-[nitro]-2β-[6'--(chloro)-pyrid-3'-yl]-cyclohexane-4-one prepared as described below are dissolved in 250 ml of ethanol, whereupon 27.0 g (0.01 mole) of stannous(II)-chloride dihydrate are added and the reaction mixture is heated to boiling for 24 hours. The solution is evaporated to dryness to yield the desired compound.

### Step 2

### (±)-1α-[Amino]-2β-[6'-(chloro)-pyrid-3'-yl]-cyclohexane-4-one-ethylene glycol

To the residue containing (±)-1α-[amino]-2β-[6'-(chloro)-pyrid-3'-yl]-cyclohexane-4-one prepared according to step 1 50 ml of ethylene glycol, 250 ml of benzene and 2.5 g of pyridinium tosylate are added. The reaction mixture is heated to boiling until no more water escapes (about 6 hours). The reaction mixture is cooled, the two layers are separated, the benzene and ethylene glycol layers are separately made alkaline by adding a concentrated ammonium hydroxide solution (to pH 9). The benzene phase is washed three times with 150 ml of saturated brine each and dried over magnesium sulfate. The ethylene glycol phase is filtered, the precipitate is thoroughly washed with 500 ml of chloroform. To the filtrate 300 ml of water are added, it is extracted five times with 200 ml of chloroform each, the combined organic phases are washed twice with 300 ml of saturated brine each and dried over magnesium sulfate. The chloroform and benzene layers are combined and evaporated. The residue is purified on a silica column by eluting with a 10:1 mixture of chloroform and methanol. Thus 1.79 g of the pure desired compound are obtained in the form of a colourless foam, yield 67%, R_{f} = 0.34.

### Step 3a

### (±)-1α -[Tosylamino]-2β-[6'-(chloro)-pyrid-3'-yl]-cyclohexane-4-one-ethylene ketale

2.0 g (0.007442 mole) of (±)-1α-[amino]-2β-[6'-(chloro)-pyrid-3'-yl]-cyclohexane-4-one-ethylene ketale prepared according to step 2 are dissolved in 60 ml of anhydrous dimethyl formamide, whereupon 2.1 ml (0.01506 mole) of triethyl amine and 2.84 g (0.01488 mole) of p-toluenesulfonyl chloride are added. The reaction mixture is stirred at room temperature for an hour and poured into 500 ml of icecold water. The precipitated product is filtered, dried and recrystallized from ethanol. Thus 2.2 g of the desired compound are obtained, yield 69.9%, mp.: 182-184°C. R_{f} = 0.36.

### Step 3b

### (±)-1α-[Ditosylamino]-2β-[6'-(chloro)-pyrid-3'-yl]-cyclohexane-4-one-ethylene ketale

4.4 g (0.0104 mole) of (±)-1α-[tosylamino]-2β-[6'-(chloro)-pyrid-3'-yl]-cyclohexane-4-one-ethylene ketale prepared according to step 3a) are dissolved in 250 ml of anhydrous dimethyl formamide, whereupon 1.5 g (0.0312 mole) of sodium hydride are added in small portions. The reaction mixture is stirred at room temperature for an hour, 5.95 g (0.0312 mole) of p-toluene-sulfonyl chloride are added and stirring is continued for 2 hours. The reaction mixture is poured into 2 l of icecold water, the precipitated product is filtered, dried and the residue is purified on a silica column by eluting with a 1:1 mixture of n-hexane and ethyl acetate. Thus 4.6 g of the pure desired compound are obtained, yield 57%, mp.: 210-213°C, R_{f} = 0.39.

### Step 4

### (±)-1α-[Ditosylamino]-2β-[6'-(chloro)-pyrid-3'-yl]-cyclohexane-4-one

4.0 g (0.006931 mole) of (±)-1α-[ditosylamino]-2β-[6'-(chloro)-pyrid-3'-yl]-cyclohexane-4-one-ethylene ketale prepared according to step 3b) are dissolved in 200 ml of acetone whereupon 10 ml of concentrated hydrochlorid acid are added. The reaction mixture is heated to boiling for 4 hours at such a rate that the acetone distilled off is continuously replaced by adding dropwise fresh acetone. The reaction mixture is cooled, dissolved in 200 ml of chloroform and the pH is adjusted to 9 by adding a 10% by weight sodium hydroxide solution. The two phases are thoroughly shaken together, the aqueous layer is extracted twice with 100 ml of chloroform each. The chloroform solution is washed twice with 200 ml of water each and once with 200 ml of saturated brine, dried over magnesium sulfate and evaporated. The dry residue is purified on a silica column with a 10:1 mixture of benzene and methanol. Thus 1.55 g of the pure desired compound are obtained in the form of a colourless foam, yield 42%, R_{f} = 0.53.

### Step 5

### (±)-4β-[Amino]-1α-[ditosylamino]-2β-[6'-(chloro)-pyrid-3'-yl]-cyclohexane

200 mg (0.3751 millimole) of (±)-1α-[ditosylamino]-2β-[6'-(chloro)-pyrid-3'-yl]-cyclohexane-4-one prepared according to step 4 are suspended in 30 ml of anhydrous methanol, whereupon 289 mg (3.751 millimoles) of ammonium acetate, 24 mg (0.3751 millimole) of sodium cyanoborohydride and 16 mg (0.3751 millimole) of lithium chloride are added. The reaction mixture is stirred at room temperature for 2 hours whereupon the excess of the reducing agent is decomposed by adding a few drops of acetone and the solution is then evaporated. The dry residue is dissolved in 20 ml of chloroform and 10 ml of water, the two phases are separated and the aqueous layer is extracted twice with 10 ml of chloroform each. The combined organic solutions are washed once with 10 ml of water and once with 10 ml of saturated brine, dried over magnesium sulfate and evaporated. The dry residue is purified on a silica column and eluted with a 5:1 mixture of chloroform and methanol. Thus 116 mg of the pure desired compound are obtained, yield 58%, mp.: 158-162°C. R_{f} = 0.18.

### Step 6

### (±)-Epibatidine

100 mg (0.1872 millimole) of (±)-4β-[amino]-1α-[ditosylamino]-2β-[6'-(chloro)-pyrid-3'-yl]-cyclohexane prepared according to step 5 are dissolved in 10 ml of anhydrous dimethylformamide, whereupon 100 mg (2.64 millimoles) of sodium borohydride are added and the reaction mixture is heated to boiling for 3 hours. After cooling the solution is poured into 25 ml of water and extracted five times with 10 ml of chloroform each. The chloroform layer is washed twice with 20 ml of water each and once with 20 ml of saturated brine, dried over magnesium sulfate and evaporated. The dry residue is purified on a silica column with a 1:1 mixture of chloroform and methanol. Thus 15 mg of the pure desired compound are obtained, yield 38%. The spectrum of the product corresponds to that of racemic epibatidine.

The starting substance (±)-1α-[nitro]-2β-[6'-(chloro)-pyrid-3'-yl]-cyclohexane-4-one could be prepared as follows.

### a)

### 1-(Bromo)-5-(nitro)-pentane-2-one

80.0 g (0.61 mole) of 1-(nitro)-pentane-4-one are dissolved in 250 ml of anhydrous methanol, whereupon 31.5 ml (0.61 mole) of bromine are quickly added under cooling with ice. The reaction mixture is stirred for a further 2 hours at a rate that the internal temperature should not exceed 40°C. To the reaction mixture 250 ml of water are added, the reaction mixture is stirred at room temperature overnight. Next morning the solution is extracted three times with 300 ml of ether each, the ethereal solution is washed with a 10% by weight aqueous sodium carbonate solution free of acid, whereupon it is washed three times with 200 ml of water each and 200 ml of a saturated sodium chloride solution, dried over calcium chloride and evaporated. The dry residue is subjected to chromatography on a silica column and eluted with a 3:1 mixture of n-hexane and ethyl acetate. Thus 70.4 g of the desired compound are obtained in the form of a faint yellow liquid, yield 55%. R_{f} = 0.30. IR_{(film)}: 2950, 1720, 640 cm⁻¹.

### b)

### [5-(Nitro)-pentane-2-one]-triphenyl-phosphonium bromide

10.25 g (0.048 mole) of the bromine compound 1-(bromo)-5-(nitro)-pentane-2-one prepared according to section a) are dissolved in 30 ml of anhydrous benzene whereupon a solution of 14.09 g (0.0537 mole) of triphenyl phosphine in 50 ml of anhydrous benzene is added dropwise. The reaction mixture is stirred at room temperature for 48 hours whereby the oily precipitate becomes crystalline. The precipitated salt is filtered and washed with n-hexane. Thus 20.5 g of the desired compound are obtained,
yield 89%, mp.: 70-72°C.

### c)

### [5-(Nitro)-pentane-2-one]-triphenyl-phosphorane

8.1 g (0.0171 mole) of the phosphonium salt [5-(nitro)-pentane-2-one]-triphenyl-phosphonium bromide prepared according to section b) are dissolved in 160 ml of dichloromethane and the solution formed is stirred with 136 ml (0.0542 mole) of a 1% by weight aqueous sodium hydroxide solution for 30 minutes. The two phases are separated, the dichloromethane layer is washed three times with 100 ml of water each and with 100 ml of a saturated sodium chloride solution, dried over calcium chloride and evaporated. The dry residue is thoroughly triturated with n-hexane. Thus 4.8 g of the desired compound are obtained, yield 72%, mp.: 94-97°C.

### d)

### 1-[6'-(Chloro)-pyrid-3'-yl]-3-[oxo]-6-[nitro]-hexa-1-ene

To a solution of the 13.5 g (0.0344 mole) of the [5-(nitro)-pentane-2-one]-triphenyl-phosphorane prepared according to section c) in 70 ml of anhydrous dichloromethane a solution of 3.1 g (0.022 mole) of 6-(chloro)-pyridine-3-aldehyde in 70 ml of anhydrous dichloromethane is added. The reaction mixture is heated to boiling for 8 hours in argon atmosphere. The reaction mixture is cooled, the dichloromethane solution is washed subsequently three times with 150 ml of water each and 150 ml of a saturated sodium chloride solution, dried over calcium chloride and evaporated. The dry residue is subjected to chromatography on a silica column and eluted with a 1:1 mixture of n-hexane and ethyl acetate. Thus 4.7 g of the pure desired compound are obtained, yield 84%. Mp.: 97-100°C. R_{f} = 0.52.
IR_{(KBr)}: 1700, 1680, 1620, 1580, 1550, 1100 cm⁻¹.

### e)

### (±)-1α-[Nitro]-2β-[6'-(chloro)-pyrid-3'-yl]-cyclohexane-4-one

1.6 g (0.0063 mole) of 1-[6'-(chloro)-pyrid-3'-yl]-3-[oxo]-6-[nitro]-hexa-1-ene prepared according to section d) are dissolved in 50 ml of anhydrous tetrahydrofurane whereupon 4.0 g (0.089 mole) of potassium fluoride precipitated on aluminum oxide containing 0.0117 mole of potassium fluoride are added. The reaction mixture is stirred at room temperature overnight. The solid product is filtered, washed with 300 ml of ethyl acetate. The combined filtrates are dried over calcium chloride and evaporated. The residue is purified by chromatography on a silica column and elution with a 1:1 mixture of n-hexane and ethyl acetate. Thus 1.1 g of the pure desired product are obtained, yield 59%.
Mp.: 118-121°C.
R_{f} = 0.38. IR _{(KBr)}: 1710, 1585, 1550, 1100 cm⁻¹.

### Example 3

### Step 1

### (-)-1α-[Nitro]-2β-[6'-(chloro)-pyrid-3'-yl]-cyclohexane-4-one

5.0 g (0.01963 mole) of 1-[6'-(chloro)-pyrid-3'-yl]-3-[oxo]-6-[nitro]-hexa-1-ene prepared as described in the preparation of the starting substance of Example 1, sections a) to d) are dissolved in 50 ml of anhydrous tetrahydrofurane, whereupon 12.6 ml (0.09815 mole) of (+)-α-(phenyl)-ethyl-amine are added. The reaction mixture is allowed to stand at room temperature for 3 days, whereupon the solution is evaporated. The product is purified by subjecting the crude product to chromatography on a silica column and eluting first with a 10:1 mixture of benzene and methanol (R_{f} = 0.38) and thereafter with a 1:1 mixture of n-hexane and ethyl acetate (R_{f} = 0.42). The product is crystallized from ethanol. Thus 1.45 g of the pure desired compound are obtained, yield 29%, mp.: 149-151°C, (α)$\frac{\text{ν}}{\text{0}}$ = -86.2° (c=2, chloroform).

### Further steps

The (-)-1α-[nitro]-2β-[6'-(chloro)-pyrid-3'-yl]-cyclohexane-4-one prepared according to step 1 was converted into the corresponding epibatidine by working analogously to Example 1 starting with reacting the former instead of (±)-1α-[nitro]-2β-[6'-(chloro)-pyrid-3'-yl]-cyclohexane-4-one.

### Example 4

### A)

### (-)-Epibatidine and (+)-epibatidine dihydrochloride

### Step 1

### (+)-Epi-epibatidine and (+)-epi-epibatidine dihydrochloride

1.00 g (5 millimoles) of racemic epi-epibatidine prepared according to Example 1 are dissolved in 20 ml of hot acetone, whereupon a solution of 0.96 g (2.5 millimoles) of (-)-di-0,0'-p-toluoyl-L-tartaric acid in a mixture of 20 ml of acetone and 10 ml of water is added. The solution remains clear for some minutes but later the precipitation of crystals starts from the hot solution. The mixture is allowed to cool to room temperature and allowed to stand overnight in a refrigerator. Next morning the precipitated crystals are filtered, washed with a mixture of 5 ml of acetone and 1 ml of water and dried. Thus 1.053 g of the salt are obtained. Mp.: 188-190°C, [α]_{D}²⁵ = -56.9°
(c = 0.5, methanol).

1.00 g of the above salt is dissolved in a hot mixture of 100 ml of ethanol and 5 ml of water and the solution is allowed to stand at room temperature for a weed-end. The precipitated crystals are filtered, washed with a mixture of 5 ml of ethanol and 0.5 ml of water and dried. Thus 341 mg of the salt are obtained, mp.: 200-201°C, [α]_{D}²⁵ = -53.4° (c = 0.5, methanol).

300 mg of the above salt are suspended in a mixture of 120 ml of chloroform and 8 ml of water at room temperature and sufficient amount of a 1 molar sodium hydroxide solution (about 0.2-0.3 ml) is added to adjust the pH of the aqueous phase to 9-10. The layers are separated, the chloroform solution is washed twice with 8 ml of water each, dried over sodium sulfate, filtered and evaporated in vacuo. Thus 155 mg of an oily product are obtained, [α]_{D}²⁵ = +36.1° (c = 0.5, methanol).

### Step 2

### (-)-Epibatidine and (+)-epibatidine dihydrochloride

On epimerizating the (+)-epi-epibatidine prepared according to step 1 by boiling 20 mg (0.09 mole) of it in 3 ml of anhydrous tert.butanol in the presence of 100 mg of potassium tert.butylate for 30 hours and after evaporation purifying the product on a silica gel column (eluent: 1 : 1 mixture of benzene and methanol) (-)-epibatidine is obtained which is converted into the hydrochloride. The rotation of the salt
{[α]_{D}²⁵ = +34.8° (c = 0.36, methanol)} corresponds to the value {[α]_{D}²⁵ = +34.7° (c = 0.36, methanol)} disclosed in the Prior Art (S.R. Fletscher et al., J. Chem. Soc. Chem. Column. [1993], 1216).

### B)

### (+)-Epibatidine

### Step 1

### (-)-Epi-epibatidine

The first crystallization mother-lye obtained by the preparation of (+)-epi-epibatidine under section A) is evaporated to dryness in vacuo and the crystalline residue is made water-free by evaporation on a rotating evaporator several times. Thus 649 mg of the product are obtained, mp.: 164-176°C, [α]_{D}²⁵ = -64.8° (c = 0.5, methanol).

200 mg of the above salt are suspended in a mixture of 80 ml of chloroform and 6 ml of water. The pH is adjusted to 9-10 by adding a 1 molar sodium hydroxide solution. The layers are separated, the chloroform solution is washed with water (about 10 ml), dried over sodium sulfate, filtered and evaporated to dryness. Thus 96 mg of an oily product are obtained, [α]_{D}²⁵ = -17.2° (c = 0.5, methanol).

400 mg of the above salt {[α]_{D}²⁵ = -64.8° (c = 0.5, methanol)} are treated with 2 ml of a 1 M sodium hydroxide solution in a mixture of 150 ml of chloroform and 10 ml of water. The reaction mixture is worked up. 160 mg of an oily product are obtained. [α]_{D}²⁵ = -17.2° (c = 0.5, methanol).

The oily crude product thus obtained (160 mg, 0.76 millimoles) is dissolved in 3.2 ml of hot acetone, whereupon a solution of 153 mg (0.396 millimole) of (+)-di-0,0'-p-toluoyl-D-tartaric acid, 3.2 ml of acetone and 0.6 ml of water is added. The reaction mixture is allowed to cool to room temperature, allowed to stand for a few hours, the precipitated crystals are filtered, washed with some drops of aqueous acetone and dried. Thus 269 mg of the salt are obtained, mp.: 196-198°C, [α]_{D}²⁵ = +63.4° (c = 0.5, methanol).

200 mg of the above salt are recrystallized from a mixture of 4.5 ml of ethanol and 0.5 ml of water. Thus 124 mg of the pure salt are obtained, mp.: 204-205°C, [α]_{D}²⁵ = +60.3°, (c = 0.5, methanol).

100 mg of the salt thus obtained are treated with 2 ml of a 1 M sodium hydroxide solution in a mixture of 40 ml of chloroform and 4 ml of water. Thus 60 mg of an oily product are obtained, [α]_{D}²⁵ = -40.3° (c = 0.5, methanol).

### Step 2

### (+)-Epibatidine

On epimerizating the (-)-epi-epibatidine prepared according to step 1 analogously to section A), step 2 (+)-epibatidine is obtained. This could be converted into the (-)-epibatidine hydrochloride.

## Claims

1. Process for preparing racemic or optically active epibatidine of formula 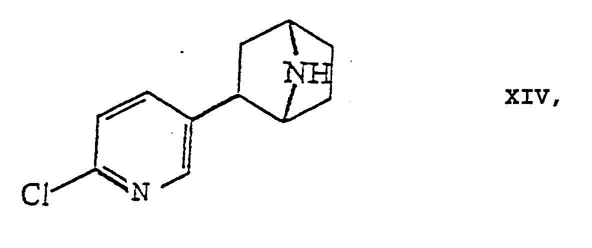 characterized by
a)
α) selectively reducing the nitro group of 1-[nitro]-2-[6'-(chloro)-pyrid-3'-yl]-cyclohexane-4-one of formula 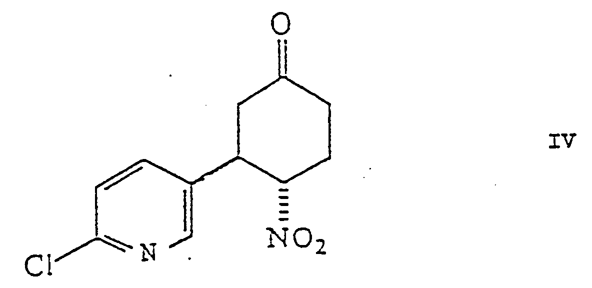 to yield 1-[amino]-2-[6'-(chloro)-pyrid-3'-yl]-cyclohexane-4-one of formula 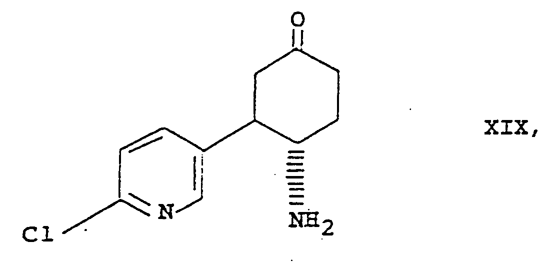
β) protecting the carbonyl group in the latter, preferably to yield a 1-[amino]-2-[6'-(chloro)-pyrid-3'-yl]-cyclohexane-4-one ketal of general formula 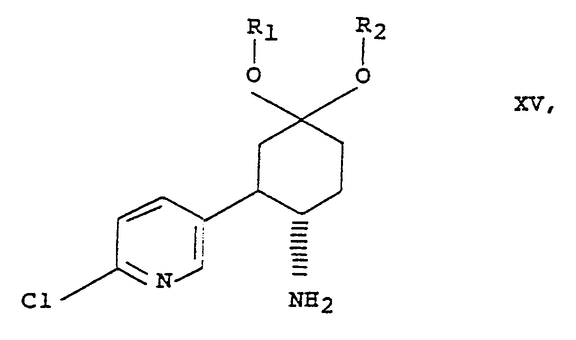 wherein
R₁ and R₂ stand for C₁₋₄-alkyl groups or together form a C₂₋₄-alkylene group,
γ) diacylating the amino group of the latter, preferably to yield a 1-[diacylamino]-2-[6'-(chloro)-pyrid-3'-yl]-cyclohexane-4-one ketal of general formula 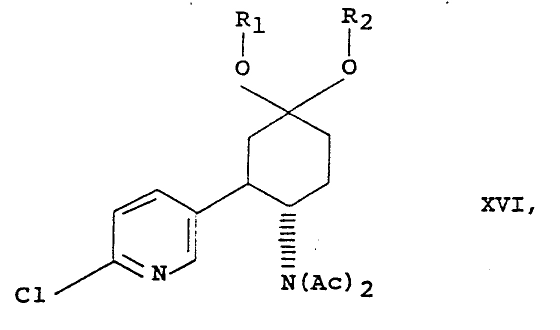 wherein
R₁ and R₂ stand for C₁₋₄-alkyl groups or together form a C₂₋₄-alkylene group and
Ac represents an acyl group,
δ) deprotecting the oxo group of the latter to yield a 1-[diacylamino]-2-[6'-(chloro)-pyrid-3'-yl]-cyclohexane-4-one of general formula 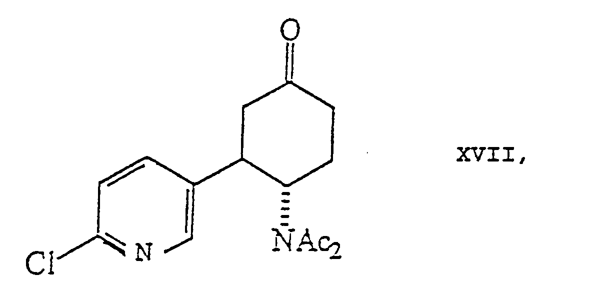 wherein
Ac stands for an acyl group,
ε) exchanging in the latter the oxo group for an amino group to yield a 1-[diacylamino]-2-[6'-(chloro)-pyrid-3'-yl]-4-[amino]-cyclohexane of general formula 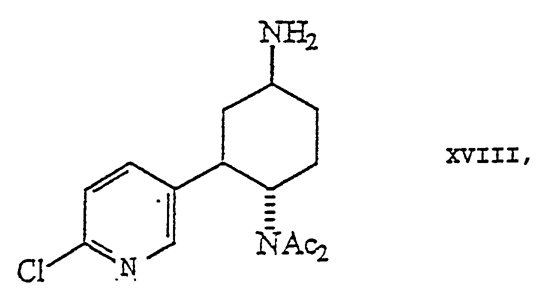 wherein
Ac stands for an acyl group,
and
ω) cyclizing the latter into epibatidine of formula XIV
or
b)
α) cyclizing 1-[6'-(chloro)-pyrid-3'-yl]-3-[oxo]-6-[nitro]-hexa-1-ene of formula 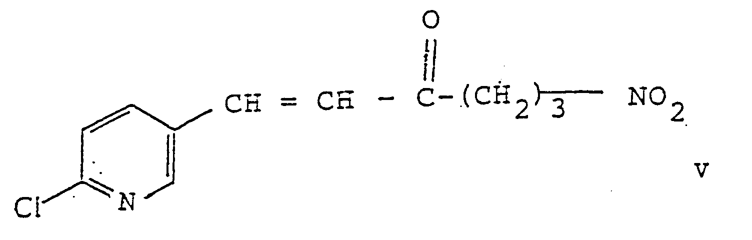 in the presence of an optically active base to optically active 1-[nitro]-2-[6'-(chloro)-pyrid-3'-yl]-cyclohexane-4-one of formula IV
and thereafter
β) converting the optically active 1-[nitro]-2-[6'-(chloro)-pyrid-3'-yl]-cyclohexane-4-one of formula IV into an optically active epibatidine of formula XIV analogously to variant a).

2. Process according to claim 1a), characterized by carrying out the selective reduction of the nitro group of 1-[nitro]-2-[6'-(chloro)-pyrid-3'-yl]-cyclohexane-4-one of formula IV by catalytic hydrogenation or reduction with a chemical agent.

3. Process according to claim 1a) or 2, characterized by carrying out the selective reduction of the nitro group of 1-[nitro]-2-[6'-(chloro)-pyrid-3'-yl]-cyclohexane-4-one of formula IV by Bechamps reduction, or with zinc in glacial acetic acid or with zinc, iron or tin in hydrochloric acid or with stannous (II)-chloride.

4. Process according to claim 1a), characterized by carrying out the protection of the carbonyl group of 1-[amino]-2-[6'-(chloro)-pyrid-3'-yl]-cyclohexane-4-one of formula XIX in the form of an ethylene ketale.

5. Process according to claim 1a), characterized by carrying out the diacylation the amino group of the 1-[amino]-2-[6'-(chloro)-pyrid-3'-yl]-cyclo hexane-4-one ketal of general formula XV to yield a 1-[diacylamino]-2-[6'-(chloro)-pyrid-3'-yl]-cyclohexane-4-one ketal of general formula XVI, in which Ac stands for C₁₋₄-alkylsulfonyl or arylsulfonyl, particularly methanesulfonyl, p-toluenesulfonyl or p-(bromo)-phenylsulfonyl.

6. Process according to claim 1a), characterized by carrying out the deprotection of the oxo group of the 1-[diacylamino]-2-[6'-(chloro)-pyrid-3'-yl]-cyclohexane-4-one ketal of general formula XVI by acidic treatment.

7. Process according to claim 1a), characterized by carrying out the exchange of the oxo group of the 1-[diacylamino]-2-[6'-(chloro)-pyrid-3'-yl]-cyclohexane-4-one of general formula XVII for an amino group by reduction with a complex metal hydride in the presence of ammonium acetate.

8. Process according to claim 1a) or 7, characterized by using for the exchange of oxo group for amino group lithium cyano borohydride and/or sodium cyanoborohydride as complex metal hydride(s).

9. Process according to claim 1a), characterized by carrying out the cyclization of the 1-[diacylamino]-2-[6'-(chloro)-pyrid-3'-yl]-4-[amino]-cyclo hexane of general formula XVIII by heating in [an] organic solvent(s).

10. Process according to claim 1a) or 9, characterized by using for the cyclization as organic solvent(s) [an] anhydrous dipolar aprotic solvent(s), most preferably dimethyl formamide.

11. Process according to claim 1a), 9 or 10, characterized by carrying out the cyclization in the presence of sodium borohydride.

12. Process according to claim 1b) α), characterized by using for the cyclization (+)-α-(phenyl)-ethyl-amine or (-)-α-(phenyl)-ethyl-amine as optically active base.
